**Europäisches Patentamt**

(19) **European Patent Office**

**Office européen des brevets**

(11) Publication number: **0 239 177 B1**

(12) # EUROPEAN PATENT SPECIFICATION

(45) Date of publication of the patent specification: **24.10.90**

(21) Application number: **87200552.5**

(22) Date of filing: **24.03.87**

(51) Int. Cl.⁵: **C07C 303/44, C07C 309/03**

(54) Process for the extraction of paraffins from mixtures thereof with alkane-sulphonic acids.

(30) Priority: **27.03.86 IT 1989386**

(43) Date of publication of application:
**30.09.87 Bulletin 87/40**

(45) Publication of the grant of the patent:
**24.10.90 Bulletin 90/43**

(84) Designated Contracting States:
**AT BE CH DE ES FR GB GR LI LU NL SE**

(56) References cited:
**FR-A- 2 376 842**
**US-A- 2 228 598**

(73) Proprietor: **ENIRICERCHE S.p.A., Corso Venezia 16, I-20121 Milan(IT)**
Proprietor: **ENICHEM AUGUSTA S.p.A., Via Ruggero Settimo 55, I-90139 Palermo(IT)**

(72) Inventor: **Faggian,Lucio, Via Kennedy 32, I-20097 San Donato Milanese Milan(IT)**
Inventor: **Marcotullio, Armando, Via Trento 4, I-20097 San Donato Milanese Milan(IT)**
Inventor: **Platone, Edoardo, Via Pace 6, I-20097 San Donato Milanese Milan(IT)**
Inventor: **Picchi, Emilio, Via Faenza 26/8, I-20142 Milan(IT)**

(74) Representative: **Roggero, Sergio et al, Ing. Barzanò & Zanardo Milano S.p.A. Via Borgonuovo 10, I-20121 Milano(IT)**

## Description

The present invention relates to a process for the extraction of paraffins from mixtures thereof with alkane-sulphonic acids.

More particularly, the present invention relates to a process for the extraction of paraffins from mixtures of said paraffins with alkane-sulphonic acids, sulphuric acid, slightly polar alcohols and water.

The mixtures of alkane-sulphonic acids and paraffins are obtained in particular by the process of sulphoxidation of n-paraffins, e.g., according to German patent 910,165.

The problems to be faced with the above said mixtures is that of the recovery of alkane-sulphonic acids or of their salts, and of paraffins, from sulphuric acid and the possible excess of $SO_2$ used in the sulphoxidation.

As to $SO_2$, no particular problems exist, in as much as a distillation under a moderate vacuum, or a stripping with oxygen, which is recycled to the sulphoxidation reactor, is enough to completely separate it from the solution.

For the separation of paraffins not converted into alkane-sulphonic acids, and of sulphuric acid, several processes have been suggested, one of which (European patent application 131,913) suggests isolating the alkane-sulphonic acids, or the alkane-sulphonates, from the mixtures obtained by sulphonating the paraffins, and subsequently separating sulphuric acid, by adding to the mixture as obtained by the sulphoxidation of n-paraffins, alcohols having a number of carbon atoms selected between 2 or 3, in such a way an upper phase, containing most of paraffins, being separated, and the mixture being then submitted to a separation of sulphuric acid by a weakly polar organic solvent immiscible, or sparingly miscible with water.

The product containing the possibly salified alkane-sulphonic acids is heated for the purpose of separating the solvent and the residual paraffins, and is optionally whitened with hydrogen peroxide.

The processes of the known art as disclosed in the above said European patent application, suffer from the drawback that the separation of paraffins is very poor when the alcohol having a number of carbon atoms selected from 2 or 3 is used, so that the subsequent heating step must be carried out for a very long time, with consequent high energy consumption, and with the danger of deteriorating the alkane-sulphonic acids or alkane-sulphonates.

It was surprisingly found that overcoming the drawbacks of the known art is possible by resorting to use of $CO_2$ in the supercritical state for the extraction of paraffins.

This invention, therefore, provides: A process for the extraction of paraffins from mixtures thereof with alkane-sulphonic acids, particularly from such mixtures as obtained by the sulphoxidation of paraffins having a number of carbon atoms from 12 to 18, characterized in that the aqueous mixture which contains the paraffins and the alkane-sulphonic acids plus sulphuric acid in the presence of one or more aliphatic alcohols, is subjected to extraction with carbon dioxide at a minimum supercritical temperature of 32°C and under a minimum supercritical pressure of 73,8 bar.

Sulphuric acid is then neutralized or eliminated by known means, so that the alkane-sulphonic acids, or their salts, are recovered.

According to a preferred embodiment of the process of the present invention, the extraction is carried out at a temperature above 32°C and not exceeding 80°C.

Still preferably, the ratio of $CO_2$ to SAS, in which SAS is a secondary alkane-sulphonate, or an alkanesulphonic acid, is from 1:1 to 50:1, on a weight basis. Yet preferably, the extraction is carried out under a pressure above 75 bar and not exceeding 350 bar.

The aliphatic alcohols having a solubility in water lower than 7% can be linear, branched or cyclic, and have a number of carbon atoms comprised within the range of from 5 to 12, they being selected, in particular, from 1-pentanol, 1-hexanol, 1-heptanol, 1-octanol, 1-decanol, 1-dodecanol, 2-ethyl-1-butanol, 2-methyl-1-pentanol, 2-ethyl-1-hexanol, 2,6-dimethyl-4-heptanol, 3-ethyl-1-hexanol, 2,7-dimethyl-octanol, 2-octanol, cyclohexanol, cyclooctanol and mixtures of these alcohols.

As regards the separation of sulphuric acid, a separating agent can be used; preferably, the same aliphatic alcohols having a solubility in water less than 7%, as above mentioned, can be used.

The process according to the present invention can be carried out as a continuous process or as a batch process, and is illustrated by the following examples in a non-limitative way.

## Example 1

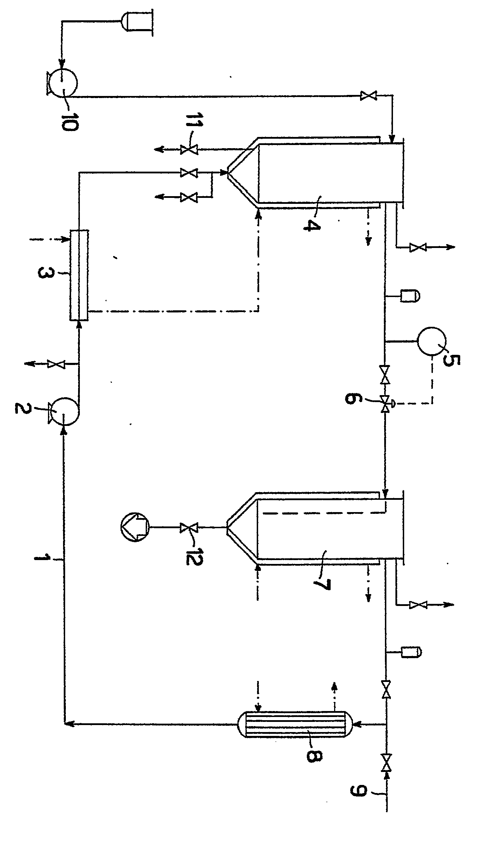

The laboratory-size extraction equipment as schematically shown in Figure 1 was used.

It consists of a refrigerating cycle for condensing $CO_2$ in heat exchanger (8). Liquid $CO_2$ (1) is pumped, by the membrane pump (2), to the pre-heater (3) and then to the extracting unit (4). The temperature of (3) and (4) is maintained constant, and at the same value, by circulating water coming from a thermostatic bath. Pressure in (4) is maintained constant at the desired value by means of the regulator (5) and of the actuation valve (6).

$CO_2$, containing the products extracted from the raw material charged to (4), outflowing from (6), leaves the supercritical field in separation unit (7), wherein $CO_2$ evaporates and is condensed in (8), to be then sent back to the already described cycle, whilst the extracted phase remains inside the separation unit. Possible make-up $CO_2$ is fed through (9).

The separation unit (7) is provided with two diametrically opposite glass windows, for the visual check of level. This latter is kept constant by acting on the temperature of water coming from a second thermostatic bath. Pressure in (7) is kept constant by means of a pressure switch, which controls the refrigerating cycle. Inside the extraction unit (4), a cylindrical vessel can be provided, which has both its cover and bottom plates made from porous sintered steel, and to which the extraction-undergoing raw product can be charged. In the preferred form, the extracting unit is packed with stainless steel bodies kept blocked by a demister.

A second pump (10) is used to deliver the raw product to be submitted to the extraction, for the continuous operating mode; in this case, the refined product is discharged through valve (11).

Inside the vessel of extraction unit (4), 126.1 g was charged of a raw mixture of $(C_{12}$-$C_{18})$-paraffin-sulphonic acids (secondary alkane-sulphonic acids = SAS), containing, besides sulphonic and disulphonic acids:

1-hexanol = 17.06% by weight
$(C_{12}$-$C_{18})$-n-paraffins = 36.72% by weight
$H_2O$ = 11.22% by weight
$H_2SO_4$ = 0.78% by weight

and underwent the extraction by supercritical $CO_2$ at 40°C and under 150 bars for 1 hour ($CO_2$/raw SAS weight ratio = 14.5).

At test end, the products contained in separation unit (7) (extracted phase) and in extraction unit (4) (refined phase) were respectively discharged through (12) and (11) and analyzed. The amount of extraction paraffin, relatively to that contained in charged raw SAS resulted to be 99.4%; that of 1-hexanol, of 91.9%.

Example 2

The equipment as described in Example 1 was used. 125 g of a raw mixture of SAS, having the composition as indicated in Example 1, was submitted to extraction by supercritical $CO_2$ at 50°C and under 150 bars for 3 hours ($CO_2$/raw SAS weight ratio = 43.8). The analyses carried out on the extracted phase and on the refined phase at test end demonstrated that paraffin was extracted to an extent of 99.53%, based on paraffin contained in charged raw SAS, and 1-hexanol to an extent of 98.8%.

Example 3

The equipment as described in Example 1 was used. 119.3 g of a raw mixture of paraffin-sulphonic acids, having the composition as indicated in Example 1, was submitted to extraction by supercritical $CO_2$ at 60°C and under 150 bars for 2 hours ($CO_2$/raw SAS weight ratio = 30.5). The analyses carried out on the extracted phase and on the refined phase at test end demonstrated that paraffin was extracted to an extent of 99.87%, based on paraffin contained in charged raw SAS, and 1-hexanol to an extent of 98.12%.

**Claims**

1. Process for the extraction of paraffins from mixtures thereof with alkane-sulphonic acids, particularly from such mixtures as obtained by the sulphoxidation of paraffins having a number of carbon atoms from 12 to 18, characterized in that the aqueous mixture which contains the paraffins and the alkane-sulphonic acids plus sulphuric acid in the presence of one or more aliphatic alcohols, is subjected to extraction with carbon dioxide at a minimum supercritical temperature of 32°C and under a minimum supercritical pressure of 73,8 bar.

2. Process according to claim 1, wherein the extraction is carried out at a temperature above 32°C and not exceeding 80°C.

3. Process according to claim 1, wherein the ratio of $CO_2$ to SAS, in which SAS is a secondary alkane-sulphonate or an alkane-sulphonic acid, is from 1:1 to 50:1, on a weight basis.

4. Process according to claim 1, wherein the extraction is carried out under a pressure above 75 bar and not exceeding 350 bar.

**Patentansprüche**

1. Verfahren zum Extrahieren von Paraffinen aus deren Mischung mit Alkansulfonsäuren, insbesondere aus solchen Mischungen, die durch Sulfoxidation von Parrafinen mit einer Kohlenstoffatomanzahl von 12 bis 18 erhalten werden, dadurch gekennzeichnet, daß die Paraffine und die Alkansulfonsäuren sowie Schwefelsäure in Gegenwart eines oder mehrerer aliphatischer Alkohole enthaltene wäßrige Gemisch einer Extraktion mit Kohlendioxid bei einer überkritischen Mindesttemperatur von 32°C und unter einem überkritischen Mindestdruck von 73,8 bar unterworfen wird.

2. Verfahren nach Anspruch 1, worin die Extraktion bei einer Temperatur oberhalb 32°C, die 80°C nicht übersteigt, ausgeführt wird.

3. Verfahren nach Anspruch 1, worin das Verhältnis von $CO_2$ zu SAS, worin SAS ein sekundäres Alkansulfonat oder eine Alkansulfonsäure bedeutet, von 1:1 bis 50:1, auf Gewichtsbasis bezogen, beträgt.

4. Verfahren nach Anspruch 1, worin die Extraktion unter einem Druck von über 75 bar, der 350 bar nicht übersteigt, ausgeführt wird.

**Revendications**

1. Procédé pour l'extraction de paraffines de leurs mélanges avec des acides alcanesulfoniques, en particulier de tels mélanges résultant de la sulfoxydation des paraffines ayant un nombre d'atomes de carbone de 12 à 18, caractérisé en ce que le mélange aqueux, qui contient les paraffines et les acides alcanesulfoniques et en outre de l'acide sulfurique en présence d'un ou plusieurs alcool(s) aliphatique(s), est soumis à une extraction avec du dioxyde de carbone à une température supercritique minimale de 32°C et sous une pression supercritique minimale de 73,8 bars.

2. Procédé selon la revendication 1, caractérisé en ce que l'extraction est effectuée à une température au-dessus de 32°C et ne dépassant pas 80°C.

3. Procédé selon la revendication 1, caractérisé en ce que le rapport en poids de $CO_2$ à SAS, SAS désignant un alcanesulfonate ou un acide alcanesulfonique secondaire, est de 1:1 à 50:1.

4. Procédé selon la revendication 1, caractérisé en ce que l'extraction est effectuée sous une pression supérieure à 75 bars et ne dépassant pas 350 bars.